# FASCICULE DE BREVET EUROPEEN

(11) **EP 2 205 284 B1**
(45) Date de publication et mention de la délivrance du brevet: **05.12.2012**
(21) Numéro de dépôt: 08842664.8
(22) Date de dépôt: 06.10.2008
(51) Int. Cl.: A61K 49/18, A61K 49/04, B82Y 5/00, B82Y 15/00

(54) **NOUVEAU PROCEDE DE PREPARATION DE NANOPARTICULES RECOUVERTES D'UNE COUCHE STABILISATRICE GEM-BISPHOSPHONATE COUPLEE A DES LIGANDS DE BIODISTRIBUTION HYDROPHILE**
NEUARTIGES VERFAHREN ZUR HERSTELLUNG VON MIT EINER AN HYDROPHILE BIODISTRIBUTIONS-LIGANDEN GEKOPPELTEN GEM-BISPHOSPHONAT-STABILISIERUNGSSCHICHT BEDECKTEN NANOPARTIKELN
NOVEL METHOD FOR PREPARING NANOPARTICLES COVERED WITH A GEM-BISPHOSPHONATE STABILISING LAYER COUPLED TO HYDROPHILIC BIODISTRIBUTION LIGANDS

(30) Priorité: 05.10.2007 FR 0758103
(43) Date de publication de la demande: 14.07.2010
(73) Titulaire: GUERBET, 93420 Villepinte (FR)
(72) Inventeur: PORT, Marc, F-95170 Deuil La Barre (FR); ROUSSEAUX, Olivier, F-60300 Senlis (FR)
(74) Mandataire: Jacobson, Claude
(86) Numéro de dépôt international: PCT/FR2008/051802
(87) Numéro de publication internationale: WO 2009/053596

(56) Documents cités:
- WO-A-2004/058275
- WO-A-2009/053597
- US-A- 5 916 539
- US-A1- 2003 229 280
- WANG, LING ET AL: "A Biocompatible Method of Decorporation: Bisphosphonate-Modified Magnetite Nanoparticles to Remove Uranyl Ions from Blood" JOURNAL OF THE AMERICAN CHEMICAL SOCIETY , 128(41), 13358-13359 CODEN: JACSAT; ISSN: 0002-7863, 2006, XP002475442
- PORTET D ET AL: "NONPOLYMERIC COATINGS OF IRON OXIDE COLLOIDS FOR BIOLOGICAL USE AS MAGNETIC RESONANCE IMAGING CONTRAST AGENTS" JOURNAL OF COLLOID AND INTERFACE SCIENCE, ACADEMIC PRESS, NEW YORK, NY, US, vol. 238, no. 1, 1 juillet 2001 (2001-07-01), pages 37-42, XP001162799 ISSN: 0021-9797

## Description

L'invention concerne un nouveau procédé de préparation de nanoparticules pour l'imagerie médicale comprenant un noyau métallique, une couche stabilisatrice organique et au moins un ligand de ciblage d'un tissu pathologique.

On connaît des nanoparticules métalliques utilisées en imagerie de diagnostique, notamment en imagerie par résonance magnétique IRM, comportant un noyau métallique, recouvert d'une couche organique stabilisatrice couplée le cas échéant à des ligands de ciblage biologique.

Parmi ces nanoparticules, on connaît notamment des nanoparticules métalliques couramment désignées USPIO qui sont de très petites particules d'oxyde de fer, dont notamment de magnétite (Fe₃O₄), de maghémite (γ-Fe₂O₃) et autres composés minéraux magnétiques d'éléments de transition, de taille inférieure à environ 100-150 nm.

Pour obtenir des solutions colloïdales de particules magnétiques, stables en milieu physiologique, il est nécessaire de conditionner la surface des particules magnétiques. Pour cela, on recouvre la particule d'une couche organique stabilisatrice constituée de macromolécules telles que des carbohydrates comme le dextran, ou de petites molécules organiques telles que des acides carboxyliques.

Afin d'obtenir des informations pertinentes pour le diagnostic par imagerie médicale, il est très avantageux de coupler ces dernières à des ligands de ciblage appropriés, de manière à ce que les particules se lient à et/ou soient reconnues par des cellules ou tissus cibles. Cette reconnaissance peut être avantageusement assurée à l'aide de ligands ayant un effet sur la biodistribution du produit, par exemple par un mécanisme de type phagocytose de la particule par des cellules du système immunitaire telles que les macrophages. Ces ligands sont par exemple des groupements hydrophiles tels que des groupements aminoalcool, ou des composés de type polyéthylène glycol (PEG).

Le document WO 2004/058275 décrit la synthèse de composés utilisant, comme couche de stabilisation/d'attache, une couche de type *gem-*bisphosphonate, et comme ligand, parmi les nombreux ligands possibles, des groupements hydrophiles à effet sur la biodistribution (ligands de biodistribution). Il est en particulier décrit des composés se présentant sous la forme d'un noyau N métallique recouverts d'éléments de ciblage de formule S-C, dans laquelle :
- S est un groupement *gem*-bisphosphonate greffé au noyau et de formule (I)

   X-L-CH(PO₃H₂)₂ (I)
- C est un ligand hydrophile (couplée à la fonction X) de type aminoalcool et/ou de type PEG ;
avec :
● L représente un groupement organique reliant la fonction X à la fonction *gem*-bisphosphonate -CH(PO₃H₂)₂ ;
● X représente une fonction chimique capable d'être couplée au ligand hydrophile C.

Pour la préparation de ces composés, le procédé antérieur (utilisé de manière générale pour les USPIO) comprend schématiquement les étapes suivantes :
- préparation du noyau métallique N des nanoparticules métalliques
- enrobage du noyau N avec la couche stabilisatrice *gem-*bisphosphonate de formule

   X-L-CH(PO₃H₂)₂ (I)
- couplage de la particule obtenue avec le ou les groupes hydrophiles.

On cherche à améliorer ce procédé pour obtenir des composés dont la quantité de ligand greffée est parfaitement maîtrisée, afin d'optimiser et de contrôler avec reproductibilité le taux de greffage sur le noyau, d'éviter des étapes de purification et de simplifier la maîtrise pharmaceutique en minimisant les risques de contamination bactérienne ou pyrogène, et ainsi d'obtenir une fabrication du produit performante au niveau industriel.

Par ailleurs, dans le cas notamment de ligands de biodistribution de type aminoalcool ou polyéthylèneglycol, décrits plus loin en détail, un problème supplémentaire réside dans la quantité nécessaire de ligand à utiliser dans le procédé décrit dans WO2004058275. En effet, pour obtenir au final un taux de couverture en éléments de ciblage S-C, avantageusement élevé, notamment un taux de couverture de plus de 80% des sites de fixation possibles sur le noyau, il fallait dans ce procédé antérieur utiliser une quantité importante de ligand C en excès (il fallait ajouter, à un équivalent de composé N-S [noyau + couverture gembisphosphonate], de l'ordre de 5 équivalents de ligand hydrophile C), d'où des prix de revient industriels élevés. Ce problème était particulièrement utile à résoudre pour des aminoalcools complexes et coûteux tels que ceux de formule (IV), notamment les aminoalcools AAG1 et ses dérivés décrits ci-après dans la présente demande.

Le demandeur a réussi à résoudre ces problèmes techniques grâce à un procédé (désigné voie inverse) de préparation selon lequel on prépare des éléments constitués par un ou plusieurs ligands hydrophiles couplés chimiquement avec des groupements organiques de liaison gem-bisphosphonate, puis ces éléments [groupement de liaison-ligand] sont couplés aux nanoparticules métalliques. Les groupements organiques de liaison appartiendront ou formeront la couche stabilisatrice (ou d'attache).

A cet effet, l'invention concerne un procédé de préparation de nanoparticules métalliques, pour l'imagerie médicale notamment, comprenant un noyau métallique N recouvert d'une couche stabilisatrice organique couplée à au moins ligand hydrophile à effet sur la stabilité/biodistribution des nanoparticules, le procédé comprenant les étapes de :
a) préparation du noyau métallique N des nanoparticules métalliques;
b) préparation d'éléments de ciblage/stabilisation de formule S-C dans laquelle :
   - S est un groupe d'attache gem-bisphosphonate de formule X-L-CH(PO₃H₂)₂;
   - C est un ligand de biodistribution hydrophile choisi parmi les aminoalcools ou les PEG tels que mentionnés dans la revendication 1;
c) greffage sur le noyau N d'au moins un élément de ciblage.

Par souci de simplicité, dans la demande, on utilisera pour l'expression éléments de ciblage/stabilisation, l'expression "éléments de ciblage".

Par l'expression "greffage sur le noyau N d'au moins un élément de ciblage", on entend que l'on greffe des ensembles de ciblage de même structure, ou plusieurs ensembles de ciblage dont les groupes S et/ou les groupes C n'ont pas la même formule.

L'avantage d'avoir des groupes S différents est notamment de pouvoir augmenter ou réduire la taille hydrodynamique de l'agent de contraste (en faisant varier la taille des groupes L), ce qui permet de contribuer à optimiser la biodistribution du produit en fonction de l'indication diagnostique concernée.

Selon des réalisations, les éléments de ciblage sont des aminoalcools tels que définis dans la revendication 1.

Selon des réalisations les éléments de ciblage sont des PEG tels que définis dans la revendication 1.

Selon des réalisations, une partie des éléments de ciblage sont des aminoalcools tels que définis dans la revendication 1, et une autre partie des éléments de ciblage sont des PEG tels que définis dans la revendication 1. Les ligands peuvent être identiques ou différents entre les éléments de ciblage greffés.

Selon des réalisations, les nanoparticules métalliques obtenues après greffage auront ainsi, par exemple, 10 à 90% des éléments de ciblage avec un ligand hydrophile ayant un effet avantageux sur la biodistribution et/ou la stabilité de la particule (un aminoalcool, une branche PEG, plusieurs aminoalcools différents), et le complément (90 à 10%) des éléments de ciblage avec un ligand différent.

Selon des réalisations, on greffe sur le noyau d'une part des éléments de ciblage S-C (porteurs de groupes hydrophiles), et d'autre part des groupes de stabilisation S non porteurs de ligands de biodistribution. On aura par exemple 5 à 95% de groupes S-C et le complément (95 à 5%) de groupes S.

Le tableau en fin de description détaillée illustre différentes possibilités.

Ces taux correspondent au taux de couverture du noyau par les éléments S ou S-C (au niveau des sites de fixation possible, typiquement les sites protonés localisés à la surface de la nanoparticule). Les pourcentages sont ainsi exprimés en nombre de molécules S-C ou S par nombre de sites de fixation disponibles sur le noyau. Ainsi, pour un taux de couverture de 100%, la surface du noyau sera sensiblement totalement recouverte par des éléments S-C et/ou S (par exemple 80% de groupes S-C et 20% de groupes S). Ce taux de couverture est ainsi distinct du taux de greffage décrit ci-après.

Les nanoparticules métalliques ont un diamètre hydrodynamique de l'ordre de 10 à 100 nm, et notamment de 10 à 50 nm.

Chaque groupement S d'un ensemble de ciblage comprend au moins une partie de liaison au noyau, et au moins une fonction chimique X de couplage avec un ligand C, plus précisément de liaison covalente à une fonction réactive du ligand.

Les étapes a) et b) peuvent être réalisés dans n'importe quel ordre mais avant l'étape c).

L'ensemble des groupements S greffés sur le noyau N constitue la couche d'attache (stabilisatrice). Le taux de greffage (pourcentage en mol de composé S-C et/ou S par mol de fer ; le taux de greffage est déterminé à partir des dosages de phosphore) des éléments de ciblage S-C et/ou S sur le noyau N est typiquement compris entre 0,5 et 10%, notamment 1 à 5%, par exemple 1, 2, 3, 5, 10%, pour une taille cristalline du noyau de l'ordre de 7-8 nm.

Selon des réalisations, en plus des éléments de ciblage S-C, on greffe aussi des groupes ayant un effet sur la stabilité de la nanoparticule, par exemple des acides hydroxy mono carboxyliques, par exemple choisi parmi les suivants : acide gluconique, acide oxalique, acide mandélique, acide 4-hydroxy-3-méthoxy-mandélique, acide lactobionique, acide alpha-hydroxyhippurique, acide méthyl 2-hydroxybutyrique, acide glycolique, acide N-acétylneuraminique, acide phospho-énolpyruvique.

De manière ainsi très avantageuse, on peut parfaitement contrôler le taux de greffage des nanoparticules en composés porteurs de ligands, ce qui est très utile pour le coût, l'analyse et la caractérisation et le contrôle de l'efficacité physiologique du produit. La fabrication des éléments de ciblage S-C est par ailleurs totalement maîtrisée, en particulier leur pureté avant le greffage, ce qui est majeur pour la fabrication industrielle.

On décrit maintenant plus précisément le noyau N. Le noyau métallique des nanoparticules préparées est typiquement composé en tout ou partie d'hydroxyde de fer ; d'oxyde de fer hydraté ; d'oxydes de fer mixtes tels que des oxydes de fer mixtes de cobalt, de nickel, de manganèse, de béryllium, de magnésium, de calcium, de baryum, de strontium, de cuivre, de zinc ou de platine ; ou d'un mélange de ceux-ci. Le terme "ferrite" désigne les oxydes de fer de formule générale [xFe₂O₃,y MOz], où M désigne un métal magnétisable sous l'effet d'un champ magnétique tel que Fe, Co, Ru, Mg, Mn, le métal magnétisable pouvant être éventuellement radioactif. De façon préférentielle, les particules magnétiques des compositions de l'invention comprennent une ferrite, notamment la maghémite (γFe₂O₃) et la magnétite (Fe₃O₄), ou encore les ferrites mixtes de cobalt (Fe₂CoO₄) ou de manganèse (Fe₂MnO₄). Le noyau de la nanoparticule a été rendu acide pour faciliter le couplage des éléments S-C. Le procédé de préparation du noyau acide (avec étape utilisant l'acide nitrique) est décrit en détail dans le document WO 2004058275 (US 2004253181, notamment les paragraphe 331 à 339, page 19). Il est rappelé que ce procédé avec étape d'acidification (à pH fortement acide - typiquement entre 1 et 3) avant la fixation des groupes S et/ou S-C, permet d'obtenir des particules particulièrement avantageuses dont la polydispersité est totalement maîtrisée et qui sont en solution colloïdale stable.

On décrit maintenant les groupes L. Le groupe de liaison L est un groupement divalent, choisi parmi :
- un groupe aliphatique ; alicyclique ; alicyclique-aliphatique ; aromatique ; aromatique-aliphatique, lesdits groupes aliphatiques, alicycliques et aromatiques pouvant être éventuellement substitués par un groupe méthyle, hydroxy, méthoxy, acétoxy, amido, ou un atome de chlore, d'iode ou de brome ;
- un groupement -L₁-NHCO-L₂ où L₁ et L₂ sont soit identiques, soit différents et représentent un groupe aliphatique ; alicyclique ; aromatique ; alicyclique-aliphatique ou aromatique-aliphatique, lesdits groupes pouvant être éventuellement substitués par un groupe méthyle, hydroxy, méthoxy, acétoxy, amido ou un atome de chlore, d'iode ou de brome.

Un groupe aliphatique désigne ici une chaîne hydrocarbonée linéaire ou ramifiée comportant de préférence de 1 à 16 atomes de carbone, mieux encore de 1 à 6 atomes de carbone. De préférence, le groupe aliphatique désigne un groupe alkyle. Des exemples en sont notamment les radicaux méthyle, éthyle, propyle, isopropyle, butyle, tert-butyle, isobutyle, pentyle et hexyle.

Le terme "alicyclique" désigne une chaîne hydrocarbonée cyclique comportant de préférence de 3 à 8 atomes de carbone, de préférence un groupe cycloalkyle. A titre d'exemple, on citera notamment le cyclopropyle et le cyclohexyle.

Le terme "aromatique" représente un groupement hydrocarboné mono- ou polycyclique aromatique comprenant préférentiellement de 5 à 20 atomes de carbone, mieux encore de 6 à 18. Des exemples en sont notamment les radicaux phényles et 1- ou 2-naphtyles. Selon une variante particulière, un groupe "aromatique" au sens de l'invention peut intégrer un ou plusieurs hétéroatomes tels que le soufre, l'oxygène ou l'azote. Dans ce cas particulier, le groupe "aromatique" désigne un groupement hétéroaromatique mono- ou polycyclique.

Les groupes "aliphatique-alicyclique" et "aliphatique-aromatique" représentent des chaînes aliphatiques répondant à la définition susmentionnée, substituées par respectivement un groupement alicyclique ou aromatique tels que définis ci-dessus. A titre d'exemple de groupe aliphatique-aromatique, on peut notamment citer le benzyle.

Selon une variante préférée, L représente un groupe phénylène éventuellement substitué, les groupements X et gem-bisphosphonates pouvant être en position ortho, méta ou para.

Selon un mode de réalisation particulièrement préféré, L représente un groupe aliphatique substitué ou non, et plus préférentiellement un groupe -(CH₂)ₚ-, où p est un entier de 1 à 5.

Selon un autre mode préférentiel, L représente un groupe L₁-CONH-L₂ et plus préférentiellement un groupe -(CH₂)ₙ-NHCO-(CH₂)ₘ- où n et m représentent un nombre entier de 0 à 5.

L'extrémité X du composé gem-bisphosphonate de formule (I) est choisie de telle sorte qu'elle soit susceptible de réagir et de former une liaison covalente avec une fonction présente sur le biovecteur. Pour plus d'informations concernant ces couplages, on pourra se référer notamment à l'ouvrage Bioconjugate techniques, Greg T. Hermanson, 1995, Publisher : Academic, San Diego, Calif.

X est choisi parmi :
- -COOH,
- -NH₂, -NCS, -NH-NH₂, -CHO, alkylpyrocarbonyle (-CO-O-CO-alk), acylazidyle (-CO-N₃), iminocarbonate (-O-C(NH)-NH₂), vinylsulfuryle (-S-CH=CH₂), pyridyldisulfuryle (-S-S-Py), haloacétyle, maléimidyle, dichlorotriazinyle, halogène,
- le groupe de formule : 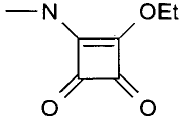
- les groupes -COOH et -NH₂ étant particulièrement préférés.

Le terme "alk" désigne au sens de la présente description un radical alkyle en C₁-C₆, le terme "Py" désignant quant à lui un radical pyridyle.

Le radical maléimidyle désigne un radical cyclique de formule :

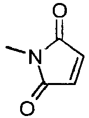

Le radical dichlorotriazinyle désigne un radical de formule :

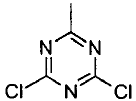

Parmi les groupes halogènes, on peut citer notamment le chlore, le brome, le fluor et l'iode, le chlore et le brome étant particulièrement préférés.

Par "haloacétyl", au sens de la présente description, on entend un radical acétyl CH₃-CO-, dont l'un des atomes d'hydrogène est substitué par un atome d'halogène, celui-ci étant tel que défini précédemment.

De préférence, X représente un groupe -COOH ou -NH₂ et L un groupe aliphatique substitué ou non, mieux encore un groupe -(CH₂)ₚ-, où p est un entier de 1 à 5.

On préfère tout particulièrement le composé de formule (la)

HOOC-(CH₂)₂-CH(PO₃H₂)₂ (la)

Selon un autre mode préférentiel, L représente un groupe L₁-CONH-L₂ et plus préférentiellement un groupe -(CH₂)ₙ-NHCO-(CH₂)ₘ- où n et m représentent un nombre entier de 0 à 5 et X représente -COOH ou -NH₂.

Bien entendu, entre également dans le cadre de l'invention le couplage de la fonction X et du biovecteur de façon indirecte, c'est-à-dire via un réactif homobifonctionnel ou hétérobifonctionnel. A titre illustratif de réactif homobifonctionnel, le glutaraldéhyde peut convenir pour réaliser le couplage, par exemple, d'une fonction X = -NH₂ avec une fonction -NH₂ du biovecteur.

Selon une variante préférée de l'invention, les fonctions X forment une liaison covalente L₃ avec le biovecteur de type
-CONH-, -COO-, -NHCO-, -OCO-, -NH-CS-NH-, -C-S-, -N-NH-CO-, -CO-NH-N-, -CH₂-NH-, -N-CH₂-, -N-CS-N-, -CO-CH₂-S-, -N-CO-CH₂-S-, -N-CO-CH₂--CH₂-S-, -CH=NH-NH-, -NH-NH=CH-, -CH=N-O-, -O-N=CH- ou répondant aux formules suivantes :

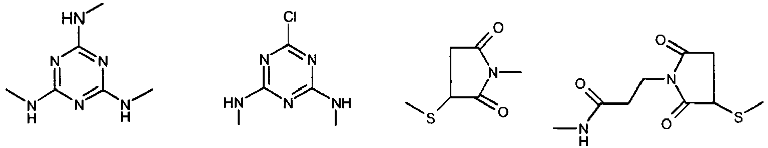

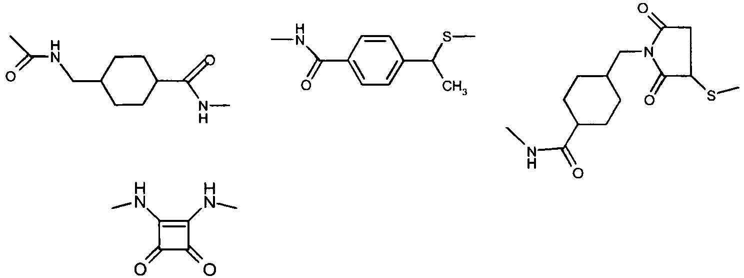

Tout ou partie, et typiquement de l'ordre de 50 à 100%, notamment 50, 60, 70, 80, 80, 95% des fonctions X du composé gem-bisphosphonate sont couplés à un ligand de biodistribution.

Le ligand de biodistribution hydrophile est un ligand aminoalcool ou polyéthylèneglycol (encore dénommé PEG), tels que définis dans la revendication 1.

Les ligands aminoalcools sont des composés de formule générale (II) : 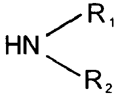 dans laquelle :
R₁ et R₂ sont identiques ou différents et représentent une chaîne hydrocarbonée aliphatique comportant de 2 à 6 atomes de carbone, substituée par 6 à 10 groupements hydroxyles, ou bien par 4 à 8 groupements hydroxyles dans le cas où R₁ et/ou R₂ est interrompu par un ou plusieurs atomes d'oxygène.

Comme exemple de ligand aminoalcool de formule (II), on peut citer notamment les ligands pour lesquels R₁ et R₂ représentent chacun indépendamment un groupe -(CH₂)-(CHOH)₄-CH₂OH ou -(CH₂)-CHOH-CH₂OH, en particulier ceux pour lesquels R₁ représente un groupe -(CH₂)-(CHOH)₄-CH₂OH ou -(CH₂)-CHOH-CH₂OH et R₂ un groupe -CH₂- (CHOH)₄-CH₂OH.

Selon un autre mode de réalisation préféré, les ligands aminoalcools sont des composés de formule (IV) 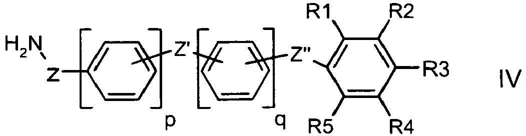 dans laquelle :
Z est une liaison, CH₂, CH₂CONH ou (CH₂)₂NHCO
Z' est une liaison, O, S, NQ, CH₂, CO, CONQ, NQCO, NQ-CONQ ou CONQCH₂CONQ,
Z" est une liaison, CONQ, NQCO ou CONQCH₂CONQ
p et q sont des nombres entiers dont la somme vaut 0 à 3 (avec selon une variante avantageuse p=q=0)
R₁, R₂, R₃, R₄ ou R₅ représentent chacun indépendamment H, Br, CI, l, CONQ₁Q₂ ou NQ₁COQ₂,
   avec Q₁ et Q₂ identiques ou différents choisis parmi H, un groupe (C₁-C₈)alkyle mono- ou polyhydroxylé et/ou éventuellement interrompu par un ou des atomes d'oxygène, de telle sorte que Q₁ et Q₂ comportent à eux deux de 4 à 10 groupes OH ;
   étant entendu qu'au moins l'un et au plus deux des groupes R₁ à R₅ représente CONQ₁Q₂ ou NQ₁COQ₂;
ou bien R₁, R₃, R₅ représentent chacun indépendamment H, Br, Cl, ou I est R₂ et R₄ représentent 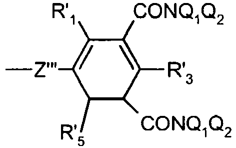 dans laquelle
   R'₁, R'₃ et R'₅, identiques ou différents, représentent H, Br, Cl ou l ;
   Q₁ et Q₂ ont la même signification que précédemment ;
   Z"' est un groupe choisi parmi CONQ, CONQCH₂CONQ, CONQCH₂, NQCONQ, CONQ(CH₂)₂NQCO ; et

Q est H ou (C₁-C₄)alkyle, lesdits groupes alkyles pouvant être linéaires ou ramifiés et étant éventuellement hydroxylés.

De préférence, Z est CH₂.

De préférence, p=q=0.

De préférence, Z" est CONH.

De préférence, R₂ et R₄ représentent CONQ₁Q₂.

De préférence, R₁, R₃, R₅ représentent Br.

De préférence, Q₁ et Q₂ représentent chacun indépendamment un groupe - (CH₂)-(CHOH)₄-CH₂OH ou -(CH₂)-CHOH-CH₂OH, en particulier un groupe -(CH₂)-(CHOH)₄-CH₂OH.

Selon un mode de réalisation particulièrement préféré, le ligand aminoalcool de formule (IV) est le composé :

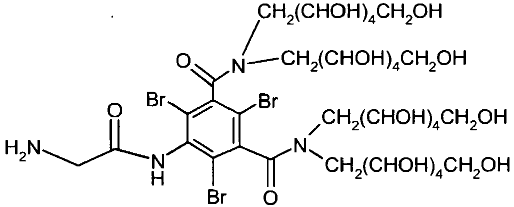

De préférence, les ligands aminoalcools selon l'invention sont couplés via leur fonction amine -NH- ou -NH₂ à la fonction X des groupes d'attache S de formule X-L-CH(PO₃H₂)₂, de sorte que les fonction hydroxyles restent libres, préservant ainsi leur caractère hydrophile.

Par « polyéthylèneglycol », au sens de la présente demande, on désigne de façon générale, des composés comprenant une chaîne -CH₂-(CH₂-O-CH₂)ₖ-CH₂OR₃ dans laquelle k varie de 2 à 100 (par exemple 2, 4, 6, 10, 50), et R₃ est choisi parmi H, alkyle ou -(CO)Alk, le terme "alkyle" ou "alk" désignant un groupe aliphatique hydrocarboné, linéaire ou ramifié, ayant environ de 1 à 6 atomes de carbone dans la chaîne.

Le terme « polyéthylèneglycol » tel qu'employé ici, englobe notamment les composés aminopolyéthylèneglycols de formule (III) : 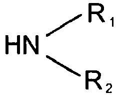 dans laquelle :
R₁ et R₂, identiques ou différents, représentent H, un groupe alkyle ou une chaîne polyéthylèneglycol de formule -CH₂-(CH₂-O-CH₂) ₖ-CH₂OR₃, étant entendu qu'au moins un des groupes R₁, R₂ représente une chaîne polyéthylèneglycol,
dans laquelle k varie de 2 à 100 (par exemple 2, 4, 6, 10, 50), et
R₃ est choisi parmi H, alkyle ou -(CO)Alk, le terme "alkyle" ou "alk" désignant un groupe aliphatique hydrocarboné, linéaire ou ramifié, ayant environ de 1 à 6 atomes de carbone dans la chaîne.

Des exemples d'aminopolyéthylèneglycols sont notamment les composés O-(2-aminoéthyl)-O'-méthylpolyéthylèneglycol 1100, O-(2-aminoéthyl)-O'-méthyl-polyéthylèneglycol 2000, O-(2-aminoéthyl)-O'-méthyl-polyéthylèneglycol 750, les composés PEG 340, PEG 750, PEG 1500, PEG2000 par exemple.

Il est précisé que le greffage des composés S-C de la demande sur le noyau N se fait par la partie CH(PO₃H₂)₂ des composés S.

Le demandeur a noté en particulier, pour les ligands aminoalcool et/ou PEG, que non seulement la synthèse est facilitée et avec un rendement amélioré, mais en plus que le produit final obtenu répond de manière très satisfaisante aux spécifications réglementaires et à l'utilisation diagnostique. La couverture de la quasi-totalité du noyau par ces groupements confère au produit une stabilité et une biodistribution particulièrement avantageuses pour ces ligands hydrophiles, notamment en améliorant la furtivité (le produit est alors avantageusement moins capté par le foie) et la captation macrophagique (le ciblage des macrophages est amélioré, avec un intérêt notamment pour le suivi des plaques d'athérome, des ganglions et autres zones d'inflammation). Par ailleurs on comprend l'avantage de la maîtrise de la quantité de ligand pour les produits à couverture mixte, par exemple ligand aminoalcool et ligand PEG, pour respecter les exigences de reproductibilité des lots de fabrication, de qualité et de sécurité pharmaceutiques.

De manière inattendue, le nouveau procédé du demandeur en voie inverse permet de diviser d'un facteur de 3 à 10 la quantité de ligand de biodistribution, en particulier de tels aminoalcools. Le tableau suivant illustre ce résultat, en prenant l'exemple du composé de l'exemple 14 utilisant comme aminoalcool le composé de l'exemple 4.

| | Voie directe (art antérieur) | Voie inverse |
|---|---|---|
| Quantité de ligand aminoalcool à ajouter pour obtenir une couverture en éléments S-C supérieure à 90 % | 5 à 10 équivalents | 1.2 à 2.2 équivalents |
| Taux de greffage (en % de ligand aminoalcool / mol de Fe) avec l'aminoalcool de l'exemple 4 | 1.2 % | 2% |
| Quantité d'aminoalcool (en mol d'aminoalcool) utilisée pour obtenir ce taux de greffage | 0,2 mol | 0,025 mol |

Ainsi, le demandeur a réussi à résoudre le double problème du contrôle du taux de greffage des ligands, et de la baisse de quantité de ligand à utiliser.

Selon un mode de réalisation particulier, le procédé selon l'invention comprend en outre le greffage d'éléments S-C-T, où C est un ligand polyéthylèneglycol et T représente un groupe chromophore.

Par « chromophore », on entend un groupement coloré, c'est-à-dire capable d'absorber l'énergie de photons dans une gamme du spectre visible tandis que les autres longueurs d'onde sont transmises ou diffusées.

Comme exemple de chromophore utile selon l'invention, on peut citer notamment le chlorhydrate de 4-(amino)fluorescéine.

L'invention concerne aussi l'utilisation des nanoparticules obtenues par le procédé du demandeur pour la préparation d'une composition diagnostique ou thérapeutique. Les nanoparticules sont en particulier utilisées comme agent de contraste de type composition de nanoparticules telles que décrites en détail dans le document WO 2004058275, pour l'imagerie IRM ou scanner RX.

Selon des réalisations, les particules sont véhiculées dans des systèmes de libération de principes actifs, tels que des systèmes d'encapsulation de type liposomes ou nanoparticules lipidiques solides qui peuvent également enfermer, en plus des nanoparticules utilisées comme agent de diagnostique, des principes actifs thérapeutiques.

L'invention a également pour objet les éléments de ciblage S-C tels que définis ci-dessus, utiles selon le procédé de l'invention.

L'invention est illustrée à l'aide des exemples détaillés suivants.

Dans ce qui suit, les abréviations M, M/L, M théorique, N et M/z, ES⁺, ES, kD et CCM ont les mêmes significations que dans le document WO 2004/058275 (US2004/253181):
M ou M/L : concentration molaire (mole/litre).
M théorique : masse moléculaire théorique.
N : normalité.
M/z : masse sur charge déterminée par spectrométrie de masse.
ES⁺ : électrospray mode positif.
ES⁻ : électrospray mode négatif.
TFA : acide trifluoroacétique.
kD : unité de masse moléculaire (kiloDalton).
CCM : Chromatographie sur Couche Mince.
Z ave : diamètre hydrodynamique mesuré par PCS.
Poly σ : polydispersité mesurée par PCS.

La nomenclature chimique qui suit est issue du logiciel ACD/NAME (société Advanced Chemistry Development Inc., Toronto, Canada), selon les règles IUPAC.

### Dosage du fer total :

Le fer est dosé par spectroscopie d'absorption atomique (Spectrophotomètre VARIAN AA10) après minéralisation par HCl concentré et dilution par rapport à une gamme étalon d'ions ferrique (0, 5, 10, 15 et 20 ppm).

### Taille des particules :

- Diamètre hydrodynamique de la particule greffée (Z ave) = Taille PCS :
   Déterminé par PCS (appareil Malvern 4700, laser 488 nm à 90°) sur un échantillon dilué à ∼ 1 millimolaire avec de l'eau PPI filtrée sur 0.22 µm.
   PCS = Photon Correlation Spectroscopy = Technique par Diffusion de Lumière Dynamique - Référence : R. Pecora dans J. of Nano. Res. (2000), 2, p. 123-131.
- Diamètre de la particule magnétique (p) (avant greffage) :
   Déterminé par déconvolution des courbes d'aimantation (mesures effectuées sur un magnétomètre SQUID) à différentes températures (férence : R.W. Chantrell dans IEEE Transactions on Magnetics (1978), 14(5), p. 975-977).

### Analyses structurales :

Par spectroscopie de masse (appareil MICROMASS VG Quattro II) avec une source Electrospray.

### Exemple 1 :

Une solution de 36 g (0,181 mole) de FeCl₂, 4H₂O, 20 ml de HCl à 37% dans 150 ml de H₂O est introduite dans un mélange constitué de 3 litres d'eau et 143 ml (0,302 mole) de FeCl₃ à 27%. 250 ml de NH₄OH à 25 % sont introduits rapidement sous forte agitation. L'ensemble est agité 30 mn. Les jus sont éliminés par décantation magnétique. Le ferrofluide est lavé 3 fois de suite avec 2 litres d'eau. Le ferrofluide nitrique est mis en agitation 15 mn avec 200 ml de HNO₃ [2M], le surnageant est éliminé par décantation magnétique. Le ferrofluide nitrique est porté à reflux avec 600 ml d'eau et 200 ml de Fe(NO₃)₃ [1 M] pendant 30 mm. Le surnageant est éliminé par décantation magnétique. Le ferrofluide nitrique est mis en agitation 15 mn avec 200 ml de HNO₃ [2M], le surnageant étant éliminé par décantation magnétique. Le ferrofluide nitrique est lavé 3 fois avec 3 litres d'acétone, puis est repris par 400 ml d'eau. La solution est évaporée sous vide jusqu'à un volume final de 250 ml.

| Concentration M/L | Z ave nm | Poly σ | Diamètre SQUID | Ms emu/cm³ |
|---|---|---|---|---|
| 4,85 | 40 nm | 0,22 | 8,5 nm | 275 |

### Exemple 2 :

108 g (0,543 mole) de FeCl₂, 4 H₂O dans 450 ml de H₂O est introduit dans une solution de 4 litres d'eau et 429ml (0,906 mole) de FeCl₃ à 27%. 750 ml de NH₄OH à 25 % sont introduits rapidement sous agitation (1200 tr/mn). L'ensemble est agité 30 mn. Les jus sont éliminés par décantation magnétique. Le ferrofluide est lavé 2 fois de suite avec 3 litres d'eau. Le ferrofluide nitrique est mis en agitation ¼ H avec 3 litres de HNO₃ [2M], le surnageant est éliminé par décantation magnétique. Le ferrofluide nitrique est porté à reflux avec 1300 ml d'eau et 700 ml de Fe(NO₃)₃ [1 M] pendant 30 mm (600 tr/mn). Le surnageant est éliminé par décantation magnétique. Le ferrofluide nitrique est mis en agitation 15 n avec 3 litres de HNO₃ [2M], le surnageant étant éliminé par décantation magnétique.

Le ferrofluide nitrique est lavé 3 fois avec 3 litres d'acétone, puis est repris par 600 ml d'eau. La solution est évaporée sous vide jusqu'à un volume final de 250 ml.

| Rendement % | Concentration M/L | Z ave (nm) | Poly σ |
|---|---|---|---|
| 81,8 | 4,45 | 31,3 | 0,21 |

200 ml de la solution précédente sont mis en agitation dans 2,4 litres de HNO₃ pendant 4 heures. Le surnageant est éliminé par décantation magnétique. Le ferrofluide nitrique est lavé 2 fois avec 3 litres d'acétone, puis est repris par 400 l d'eau. La solution est évaporée sous vide jusqu'à un volume final de 250 ml.

| Rendement % | Concentration M/L | Z ave (nm) | Poly σ |
|---|---|---|---|
| 77 | 2,742 | 23,3 | 0,20 |

### Exemple 3:

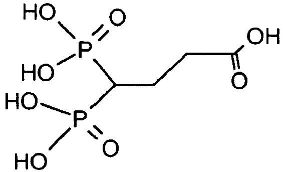

### Etape a : Diéthyl-1-[diéthoxyphosphoryl]vinylphosphonate

13 g (0,433 mole) de paraformaldéhyde et 10 ml (0,097 mole) de diéthylamine sont dissous à chaud dans 250 ml de méthanol. 24 g (8,67 10⁻² mole) de diéthyl[diéthoxy-phosphoryl]méthylphosphonate sont ensuite additionnés. L'ensemble est porté à reflux pendant 24 heures. Le milieu réactionnel est concentré sous vide. Le concentrat est repris 2 fois par 250 ml de toluène et est ensuite concentré sous vide. L'huile obtenue est dissoute dans 125 ml de toluène. 0,14 g d'acide para toluène sulfonique sont ajoutés. Le mélange est porté à reflux 24 heures avec un Dean-Stark puis est concentré à sec sous vide. Le produit est extrait avec 500 ml de CH₂Cl₂ puis est lavé 2 fois avec 250 ml d'eau. La phase organique est séchée sur MgSO₄ et concentrée sous vide. Le produit brut est purifié sur 625 g de gel de silice Merck Geduran® (40-63 µm). Elution : CH₂Cl₂ /Acétone - 50/50 (CCM - SiO₂ : Rf = 0,45)- 18,4 g sont isolés avec un rendement de 71%.
SM : M/z = 301,4 (ES+)

### Etape b: Diéthyl 2-[2.2-bis(diéthoxyphosphoryl)éthyl]malonate

1,6 g (0,01 mole) de diéthyle malonate, 0,07 g (0,001 mole) d'éthylate de sodium et 3 g (0,01 mole) de diéthyl[diéthoxy-phosphoryl]vinyl-phosphonate sont agités 15 mn dans 15 ml d'éthanol. 5 ml d'une solution saturé de NH₄Cl sont ajoutés à la solution éthanolique. L'ensemble est concentré sous vide. Le résidu est extrait avec 30 ml d'acétate d'éthyle et lavé deux fois avec 5 ml d'eau. La phase organique est séchée sur MgSO₄ puis est évaporée à sec. L'huile obtenue est purifiée sur 200 g de silice Merck Geduran^{®} ; -3,8 g sont isolés avec un rendement de 82 %.
SM : M/z 460,9 (ES+)

### Etape c : Acide 4,4-diphosphonobutanoique

7 g (15,7 10⁻² mole) de diéthyl2-[2.2-bis (diéthylphosphoryl) éthyl] malonate sont portés à reflux pendant 8 heures dans 350 ml d'HCl [5N]. L'huile brune obtenue est purifiée sur 60 g de gel de silice 60 silanisée (0,063-0,200 mm) avec une élution eau. 3,6 g sont isolés avec un rendement de 92 %.
SM: M/z 249 (ES+)

### Exemple 4 :

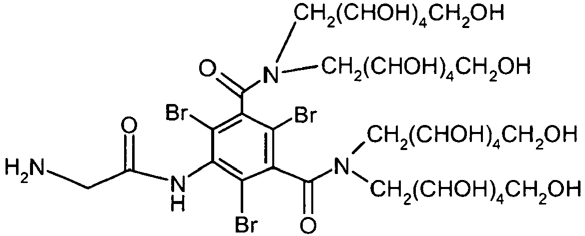

Le composé (ligand hydrophile aminoalcool) peut être préparé selon le mode opératoire décrit dans le brevet EP 0 922 700 A1.

### Exemple 5 :

600 mg du composé préparé à l'exemple 3 étape c (2,42 10⁻³ M) et 3.2 g de composé préparé à l'exemple 4 (4,85 10⁻³ M) sont dissous dans 20 ml de H₂O. Le pH est ajusté à 6.2 avec NaOH 0.1 N. 600 mg d'EDCI (3,13 10⁻³ M), 65 mg de HOBT (4,8 10⁻⁴ M) sont ajoutés et l'ensemble est agité à température ambiante pendant 24 heures. Le milieu réactionnel est versé sur 400 ml d' IPA et agité 24 heures. Le précipité est filtré puis lavé dans de l'éther éthylique et séché sous vide.

Le brut est dissous dans le minimum d'eau en ajustant le pH à 9 puis est déposé sur 150 ml de résine Amberlite Na (forme H⁺) une nuit. Le produit est élué avec de l'eau. Les bonnes fractions sont concentrées sous vide. SM, ES- : 1385.6

### Exemple 6 :

### Etape a

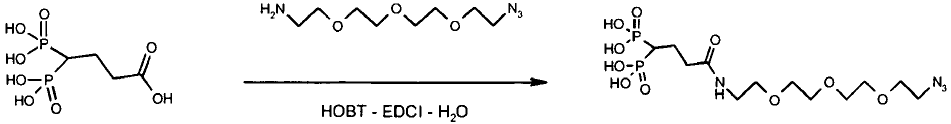

Dans un tricol de 500ml, équipé d'une électrode et d'une agitation magnétique, le gembiphosphonate (exemple 3 étape c, 30g) est dissous dans H₂O (250ml). Le pH est ramené à 5,7 par NaOH et l'amine (11-azido-3,6,9-trioxaundecan-1-amine, Fluka®, 21.8g) est ajoutée en une seule fois : l'HOBT (1,72g) puis l'EDCI (21,16g) sont ajoutés. Le milieu réactionnel est agité pendant 24 heures à température ambiante. Le milieu est évaporé jusqu'à un volume final d'environ 150ml. Le pH est ramené à 8 par NaOH. Passage de la solution sur 70ml (30 fois la théorie) de résine Amberlite 252 Na (H⁺-1.8 meq/ml) afin d'éliminer l'amino peg en excès. Elution par H₂O (V_{récupéré} = 300ml).

Evaporation jusqu'à un volume final d'environ 150ml.

Passage de la solution sur 140ml (2 fois la théorie) de résine IRA 67 (OH⁻-1.6meq/ml) afin d'éliminer les Cl⁻ en excès. Elution par H₂O (V_{récupéré} = 260ml). Evaporation jusqu'à un volume final d'environ 100ml. Passage de la solution sur 900g de silice silanisée. Elution par 2l d'H₂O puis par 2l d'un mélange H₂O/CH₃OH (50/50).
m = 28.46g, Rdt = 75%, LC/MS : en ES⁺ à m/z = 449.12.

### Etape b

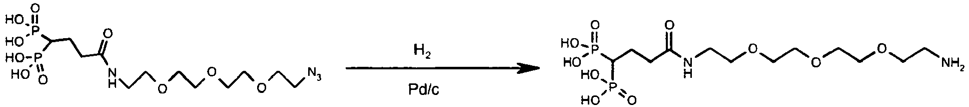

Dans le réacteur autoclave de 1L, est introduit l'azide obtenu à l'étape a (28,26g) préalablement solubilisé dans EtOH (350ml). Le milieu est acidifié par une solution d'HCl et quatre spatules de Pd/C sont introduites dans la solution. Le milieu réactionnel est agité pendant 4 heures à température ambiante sous 4 bars d'hydrogène. Le milieu réactionnel est filtré sur clarcel et la solution est évaporée à sec jusqu'à l'obtention d'une huile jaune paille (31,21g). Le produit est purifié par passage de la solution sur 200ml de résine IRA 67 (OH⁻-1.6meq/ml) afin d'éliminer les Cl⁻ en excès. Elution par H₂O.
m = 12.25g, Rdt = 46% (huile), LC/MS : en ES⁺ à m/z = 423.12.

### Etape c

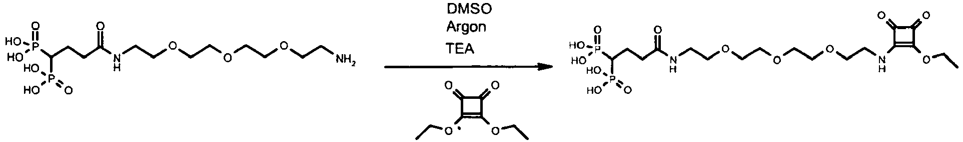

Dans un tricol de 250 ml, équipé d'une agitation magnétique, l'intermédiaire précédemment préparé (12 g) est dissous dans le DMSO (200 ml). La triéthylamine (6696 µl) puis le diéthylsquarate (4205 µl) sont ajoutés. Le milieu est agité pendant 72 heures à température ambiante. La solution est concentrée à sec à la pompe à palettes jusqu'à l'obtention d'une huile jaune qui est purifiée sur silice silanisée (Elution par 2000ml d'H₂O puis par 2000ml [H₂O /CH₃OH] (80/20) puis par 2000 ml d'un mélange H₂O/CH₃OH (50/50) et par 1000ml de CH₃OH.
m = 7.2g, Rdt = 46.4% (huile)- LC/MS : en ES⁺ à m/z = 547.25.

### Exemple 7 :

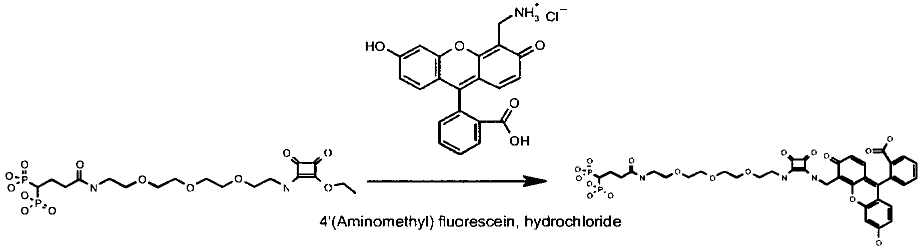

Dans un pilulier, équipé d'une électrode et d'une agitation magnétique, l'intermédiaire précédemment préparé (exemple 6 étape c) (0,137g ; 2,5*10⁻⁴ mole) est dissous dans H₂O (2ml).Le pH de la solution est ajusté à 7.5 par une solution saturée de Na₂CO₃. Le colorant (0,05g ; 1,26*10⁻⁴ mole), préalablement dissous dans du DMSO (1ml), est ajouté dans le milieu. Le pH de la solution est égal à 6,5. Le pH de la solution est ramené à 8 par une solution saturée de Na₂CO₃. La solution est agitée pendant 48H à température ambiante. Le pH de la solution est ramené à 7 par une solution d'acide chlorhydrique 1 N. La solution est évaporée à sec à la pompe à palettes. L'huile obtenue est dissoute dans H₂O (10ml) et purifiée par chromatographie sur une cartouche de silice RP18 (25-40µm) de 90g. 95 mg de produit sont isolés avec un rendement de 95%. LC/MS : en ES-m/z = 860.19

### Exemple 8:

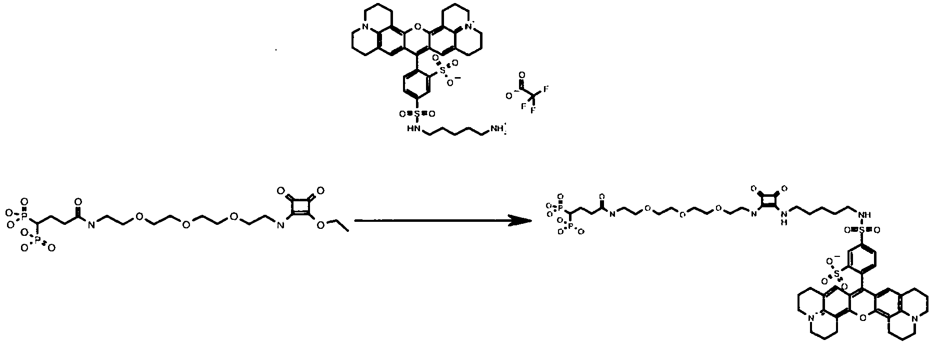

Dans un pilulier, équipé d'une électrode et d'une agitation magnétique, le composé préparé exemple 6 étape c (0.137 g, 2.5*10⁻⁴ mole) est dissous dans H₂O (2ml). Le pH de la solution est ajusté à 7.5 par une solution saturée de Na₂CO₃. Le colorant (0,102 g, 1.26*10⁻⁴ mole), préalablement dissous dans du DMSO (1 ml), est ajouté dans le milieu. Le pH de la solution est égal à 5.5. Le pH de la solution est ramené à 8 par une solution saturée de Na₂CO₃. La solution est agitée pendant 48 h à température ambiante. Le pH de la solution est ramené à 7 par une solution d'acide chlorhydrique 1 N. La solution est évaporée à sec à la pompe à palettes. L'huile obtenue est dissoute dans H₂O (10ml) et purifiée par chromatographie sur une cartouche de silice RP18 (25-40µm) de 90 g. 100 mg de produit sont isolés avec un rendement de 66%.

### Exemple 9 :

### Etape a

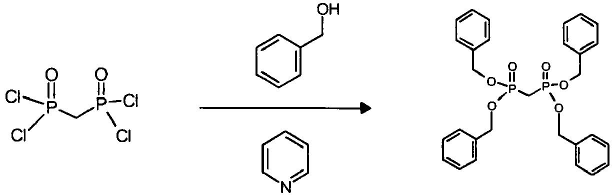

30g de Methylènebis (phosphonic dichloride) sont mis en agitation dans 180ml de toluène préalablement séché sur tamis. La température est maintenue à 0°C. Une solution de 60 ml d'alcool benzylique et d e 37,5 ml de pyridine est ajoutée goutte à goutte à l'aide d'un pousse seringue en 4 heures, la température ne devant pas dépasser les 0°C. Le milieu est agité 4 heures à TA. L'insoluble est éliminé par filtration, rincé plusieurs fois au toluène. La phase organique est lavée 3 fois avec 150ml de soude 2N, 250ml d'eau, séchée sur MgSO₄ puis concentrée. Le mélange est purifié sur silice (Eluant : Heptane/ Acétate d'éthyle : 30/70).
CCM [SiO₂ - Hept / AcOEt : 3 / 7 - R_{f} = 0.3] -Rendement : 60%
*➢ LC*/*MS* en ES+ 537.21

### Etape b

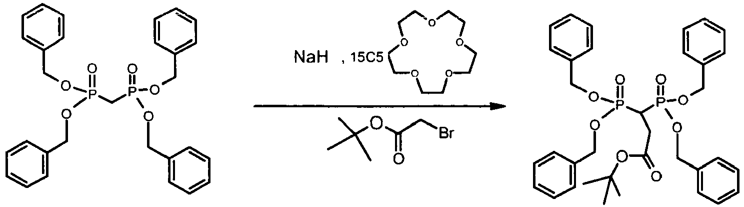

Le composé préparé à l'étape a et le 15C5 sont mis en agitation dans 240 ml de THF fraîchement distillé. 1,15 g de NaH à 60% sont ajoutés petit à petit dans le milieu. L'agitation est maintenue une ½ heure. Le t-butyl bromoacétate mis dans 25 ml de THF est ajouté goutte à goutte au bain de glace. L'ensemble est agité 3 heures à TA. Le milieu réactionnel est concentré sous vide, repris par une solution saturée de NH₄Cl et extrait par 2*200ml de CH₂Cl₂. La phase organique est séchée sur MgSO₄ et purifiée. (Cartouche Si60 ; 201 nm ; débit= 20ml/min-Gradient : CH₂Cl₂ / Acétone) -Rendement : 65%.
*➢ LClMS* en ES+ = 650.65, BP-tBu: (M+1) = 595.26

### Etape c

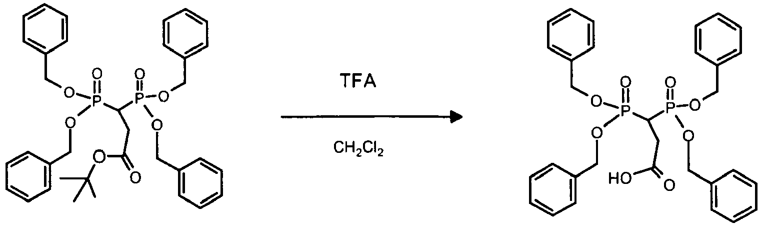

3,4g du composé issu de l'étape b sont mis en solution dans 35 ml de CH₂Cl₂. La solution est maintenue au bain de glace et 3,4 ml de TFA sont ajoutés goutte à goutte. L'ensemble est agité 4 heures à 0°C puis 20 heures à TA. Le milieu réactionnel est évaporé sous vide à 20° C. Le produit est repris avec 20ml de CH₂Cl₂ et lavé à l'eau puis purifié
(Cartouche RP 18 ; détection à 201 nm ; débit = 20 ml/min- Gradient :Eau-TFA pH=2.77/CH₃CN) - Rendement : 51%
*➢ LC*/*MS* en ES+ = 595.28

### Exemple 10 :

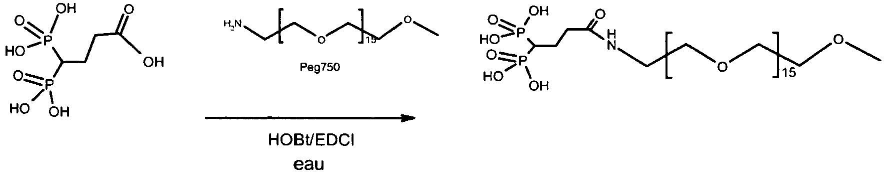

1g du composé obtenu à l'étape c de l'exemple 3 (4,03*10⁻³ mole) et 3.26 g de Peg750 (4,43*10⁻³ mole) sont dissous dans 55 ml d'eau. Le pH est ajusté à 6,2. 272 mg d'HoBt. (2,01*10⁻³ mole) sont ajoutés et le milieu réactionnel est agité 5 minutes. 1,148 g d'EDCI (6*10⁻³ mole) sont ensuite ajoutés et l'agitation maintenue 24h. Purification sur résine Amberlite 252Na avec fixation du produit à pH 9,2. 3 g sont obtenus soit un rendement de 59%.
LC/MS: Mode ES- série de pics centrée sur BP à 964.35.

### Exemple 11 :

### Etape a

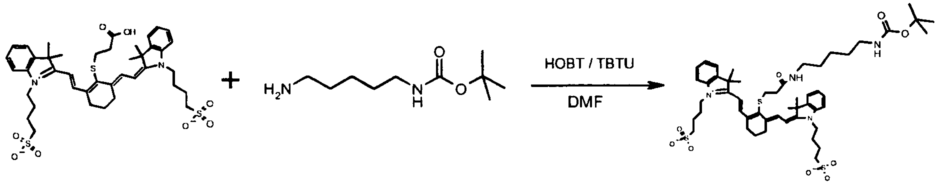

150 mg de colorant (1,88*10⁻⁴ mole, ) sont dissous dans 15 ml de DMF. Sont successivement ajoutés : 60 mg d'HoBt (4,44*10⁻⁴ mole), 51 mg de TBTU (1,58*10⁻⁴ mole), 84mg de diamine protégée ter-Butyl (4,15*10⁻⁴ mole) et 0.165 ml de DIPEA (9.4*10⁻⁴ mole).Le milieu réactionnel est agité une nuit à température ambiante. Purification par chromatoflash en phase inverse. 103,4 mg de produit sont isolés avec un rendement de 55%.
LC/MS : Mode ES+ BP à 980.89

### Etape b

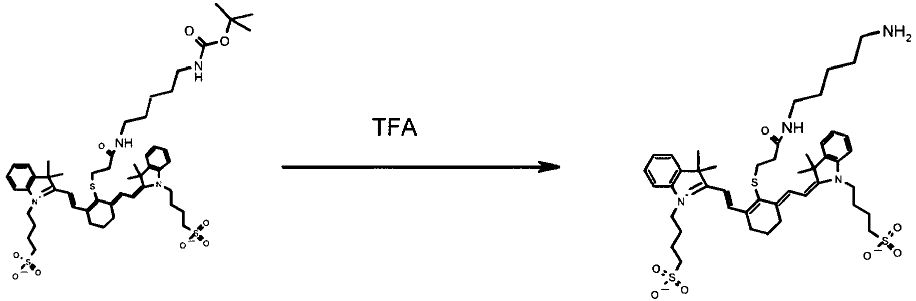

103 mg du composé préparé à l'étape **a** (1.05*10⁻⁴ mole) sont mis en agitation dans 3 ml de TFA/TIS/Eau ( proportion 90/2.5/2.5) pendant 30 minutes. Purification par chromatographie Flash phase inverse,40 mg de produit sont obtenus soit un rendement de 43%.
LC/MS : Mode Es+ BP à 879.39 z=1.

### Etape c

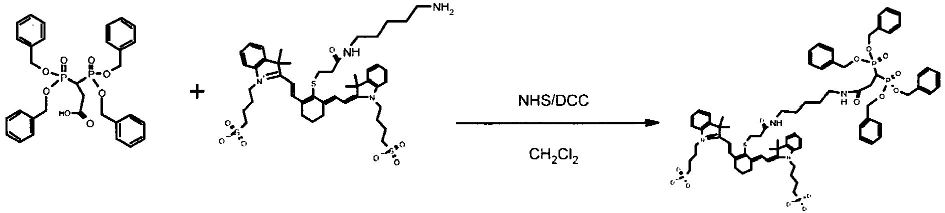

26 mg du composé obtenu à l'étape **c** de l'exemple **9** (4.37*10⁻⁵ mole), 18 mg de DCC (8,72*10⁻⁵ mole) et 8 mg de NHS (6,95*10⁻⁵ mole) sont agités 3 heures à température ambiante dans 5 ml de dichlorométhane. La DCU est filtrée. 40 mg (4,54*10⁻⁵ mole) du colorant obtenu à l'étape **b** et quelques gouttes de TEA sont alors ajoutés. L'agitation est maintenue 3 heures. Purification sur chromatographie flash phase inverse. 12 mg sont isolés avec un rendement de 20%.
LC/MS : Mode ES+ BP à 1457.33

### Etape d

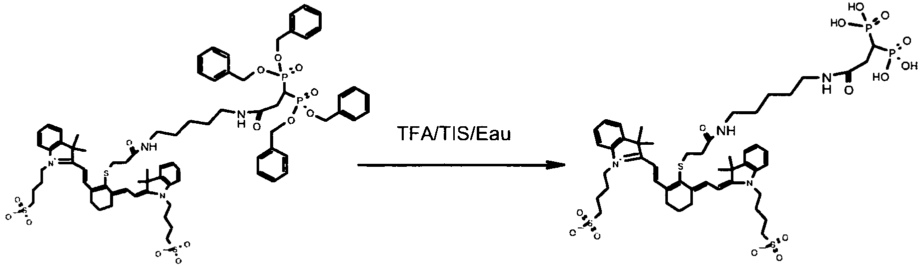

150 mg du composé préparé à l'étape précédente (1.029*10⁻⁴ mole) sont agités dans 4 ml de TFA[TIS/Eau (proportions 95/2,5/2.5). L'agitation est maintenue 3 heures à température ambiante. Purification par chromatographie flash phase inverse. 50 mg obtenus soit un rendement de 45%.
LC/MS : Mode ES- BP à 1094.53 ( z=1)

### Exemple 12 :

### Etape a

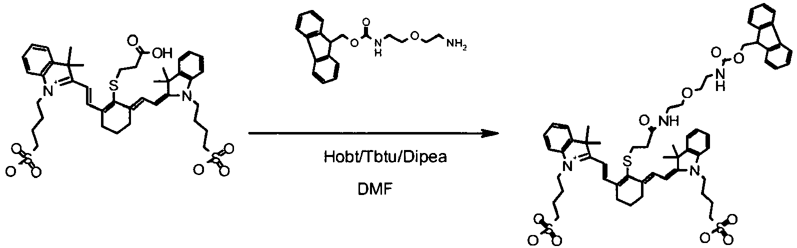

200 mg de colorant (2,51*10⁻⁴ mole) sont solubilisés dans 20 ml de DMF. Sont successivement ajoutés : 80 mg d'HoBt (5,92*10⁻⁴ mole), 68 mg de TBTU (2,11*10⁻⁴ mole), 0,220 ml de DIPEA (1,255*10⁻³ mole) et 108 mg de Fmoc-aminoethoxyéthylamine (3,30*10⁻⁴ mole). Le milieu réactionnel est agité une nuit à température ambiante. Purification sur Flash Eau/CH3CN.178 mg de produit sont isolés avec un rendement de 65%
LC/MS : Mode Es+ BP à 1103.43 (z=1)

### Etape b

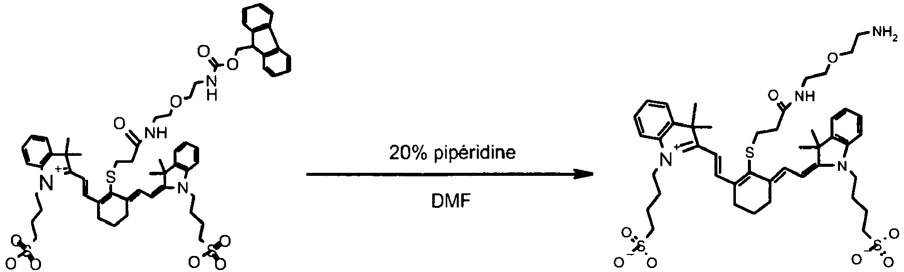

80 mg du composé préparé à l'étape précédente (9.0*10⁻⁵ mole) sont mis en solution dans 6 ml de DMF contenant de la pipéridine 20%. L'agitation est maintenue 1 heure à température ambiante. Purification par chromatographie flash phase inverse. 50 mg de produit sont obtenus soit un rendement de 40%.
LC/MS : Mode Es- BP à 880.09 ( z=1)

### Etape c

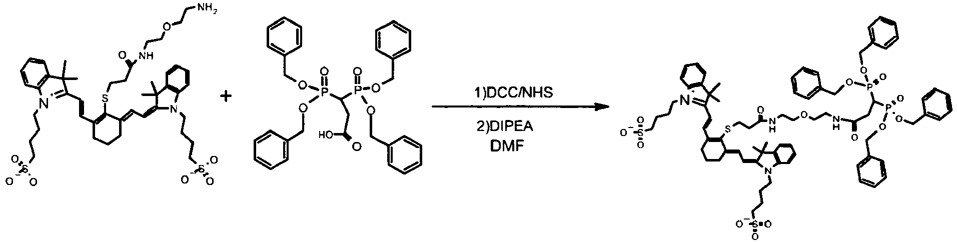

20 mg de pince benzylée obtenue à l'étape c de l'exemple **9** (3,36*10⁻⁵ mole), 14 mg de DCC (6,78*10⁻⁵ mole) et 6 mg d'NHS (5,21*10⁻⁵ mole) sont dissous dans le DMF et agités pendant 3heures à température ambiante. La DCU est éliminée. 30 mg (3,4*10⁻⁵ mole) de colorant obtenu à l'étape **b** et 17µl de DIPEA (1,02*10⁻⁴ mole) sont dissous dans 1 ml de DMF ; l'ester activé est alors ajouté goutte à goutte. L'agitation est maintenue 3 heures à température ambiante. Purification par chromatoflash en phase inverse. 30 mg sont obtenus soit un rendement de 36%.
LC/MS : Mode Es+ BP à 1458.90 ( z=1)

### Etape d

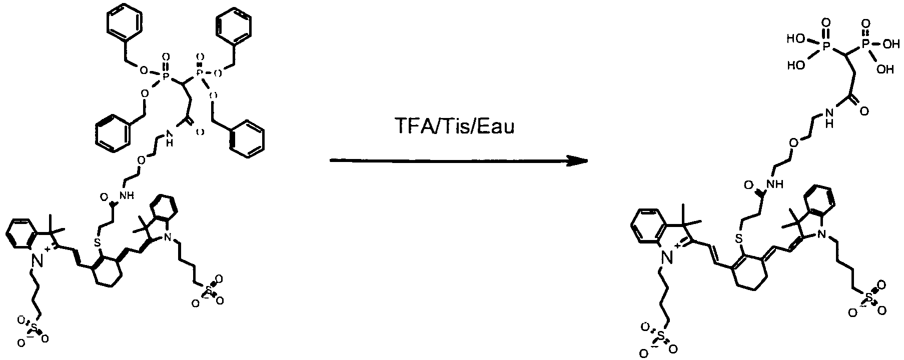

30 mg du composé préparé à l'étape **c** (2,03*10⁻⁵ mole) sont dissous dans 3 ml de TFA/TIS/Eau (proportions 95/2,5/2,5) pendant 3h50 à température ambiante. Purification par chromatoflash, 6 mg obtenus soit un rendement de 35%.
LC/MS : Mode Es+ BP à 1098.21 ( z=1) et 550.3 ( z = 2).

### Exemple13 :

### Etape a

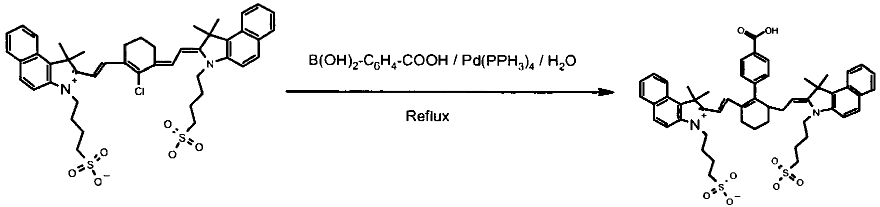

50 mg d'IR820 (Aldrich®, 5,88*10⁻⁵ mole) et 14.65 mg (8,82*10⁻⁵ mole) d'acide phénylboronique sont chauffés à reflux en présence de 10,7 mg (9,24*10⁻⁶ mole) de tetrakis(triphénylphosphine)palladium et de 40,7 mg de K2CO3 (2,9*10⁻⁴ mole) pendant 24 h à 110°C. En fin de manipulation, le palladium est filtré. Purification par chromatoflash en phase inverse, 33 mg obtenus soit un rendement de 62%.
LC/MS : Mode Es+ BP à 913.27 ( z=1) .

### Etape b

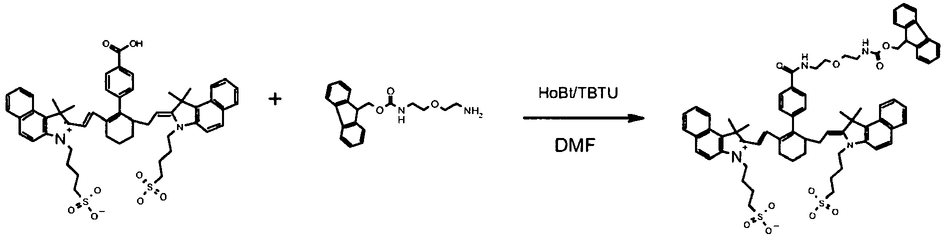

18 mg du composé préparé à l'étape **a** (1.96*10-5 mole), 7 mg d'HoBt (4.31*10-5 mole), 7 mg de TBTU (4.70*10-5 mole) 10 mg de Fmoc-aminoéthoxyéthylamine (2.15*10-6 mole) et 11µl de DIPEA (9.8*10-5 mole) sont mis en agitation une nuit à température ambiante. Le milieu réactionnel est trituré dans l'éther diéthylique (20 mg) et filtré. 13 mg de produit sont ainsi isolés avec un rendement de 55%.

### Etape c

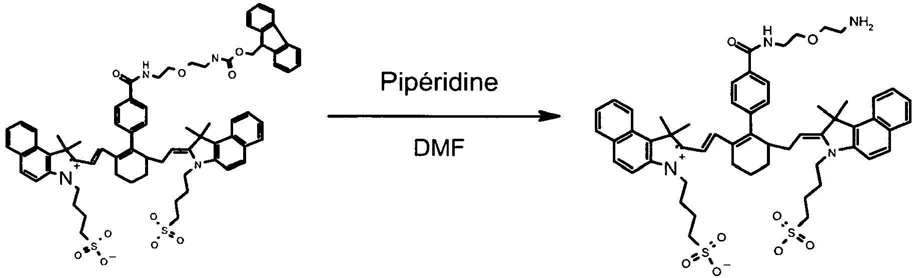

13 mg du composé obtenu à l'étape **b** (1.06*10⁻⁵ mole) sont mis en agitation dans le DMF et pipéridine 20%. L'agitation est maintenue 30 minutes à température ambiante. Précipitation dans 20 ml d'éther éthylique et agitation 1 heure à température ambiante. 4.3 mg sont obtenus soit un rendement de 40%.

### Etape d

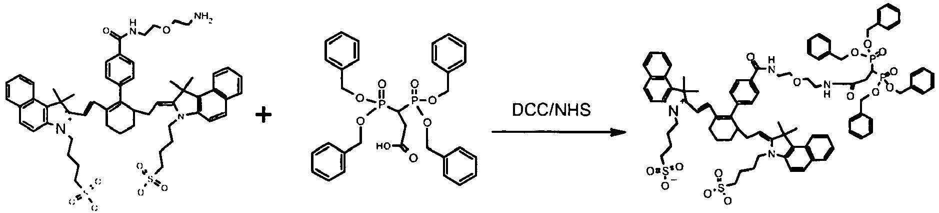

20 mg du composé obtenu à l'étape c de l'exemple **9** (3.36*10⁻⁵ mole), 14 mg de DCC (6.78*10⁻⁵ mole) et 6 mg d'NHS (5.21*10⁻⁵ mole) sont dissous pendant 3 heures à température ambiante. La DCU est éliminée. 35 mg (3.4*10⁻⁵ mole) de colorant obtenu à l'étape **c** et 17µl de DIPEA (1.02*10⁻⁴ mole) sont dissous dans 1 ml de DMF ; l'ester activé est alors ajouté goutte à goutte. L'agitation est maintenue 3 heures à température ambiante.

Précipitation dans 50 ml d'éther éthylique. 20 mg sont obtenus soit un rendement de 37%.

### Etape e

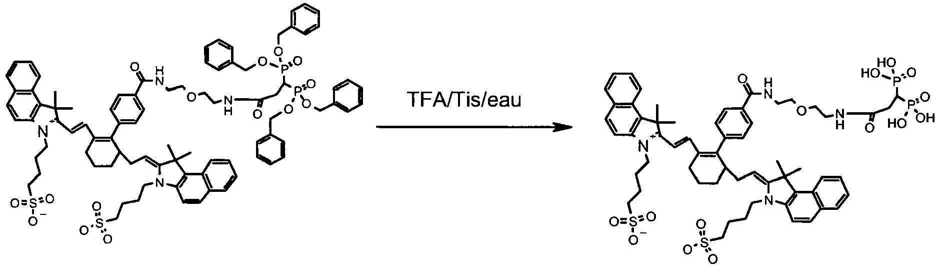

20 mg du composé préparé à l'étape **d** (1.25*10⁻⁵ mole) sont mis en agitation dans 3 ml de TFA/TIS/Eau (95/2.5/2.5) pendant 3h et à température ambiante. Purification par chromatoflash en phase inverse. 4 mg sont isolés soit un rendement de 26%.

### Exemple 14 :

60 µmoles du composé obtenu à l'exemple **5** en solution dans 10 ml d'eau sont ajoutés goutte à goutte à une solution de 1 ml de l'exemple **2** (ferrofluide acide) à 2,75 M/L dilué dans 100 ml d'eau. L'ensemble est agité pendant 20 minutes à température ambiante et le pH est ajusté à 7,2. La solution obtenue est ultrafiltrée sur une membrane ayant un seuil de coupure de 30 kD. 300 ml de filtrat sont éliminés pour obtenir une solution finale de 10 ml.
[Fe] = 0,260 M/L Taille PCS = 28 nm
Taux de greffage [composé Aminoalcool/Fe] = 2 % mole/mole

### Exemple 15 :

3 ml du composé décrit à l'exemple **2** ([Fe]= 1.336 mol/l) sont dilués dans 100 ml d'eau. A cette solution sont ajoutés successivement et avec un délais de 15 minutes entre chaque ajout, une solution de 46 mg du composé décrit à l'exemple **5** dans 2ml d'eau, une solution de 3,85 mg du composé décrit à l'exemple **7** dans 2 ml d'eau et finalement une solution de 46 mg du composé de l'exemple **5** dans 2 ml d'eau. La solution est agitée 15 minutes à température ambiante et le pH est ajusté à 7.4 avec une solution de NaOH. Le milieu est ultrafiltré sur une membrane de 30KD et le volume de la solution est ramené à 20 ml pour une concentration en fer de 0.191 mol/l. PCS : 26.8.

### Exemple 16 :

Selon le protocole de l'exemple **16,** différentes combinaisons binaires ou ternaires de composés biphosphonates dans des proportions variables sont fixées sur les particules d'oxyde de fer décrites à l'exemple **1** ou **2** tel que résumé dans le tableau suivant :

| N° | particules | Biphosphonate 1 (% mol) | Biphosphonate 2 (% mol) | Biphosphonate 3 (% mol) | Taille PCS nm |
|---|---|---|---|---|---|
| 1 | Exemple 1 | Exemple 5 (40) | Exemple 3 (60) | - | 42 |
| 2 | Exemple 2 | Exemple 5 (60) | Exemple 3 (30) | Exemple 7 (10) | 28 |
| 3 | Exemple 2 | Exemple 5 (95) | Exemple 7 (5) | - | 27 |
| 4 | Exemple 2 | Exemple 5 (90) | Exemple 8 (10) | - | 26 |
| 5 | Exemple 2 | Exemple 5 (95) | Exemple 11 (5) | - | 28 |
| 6 | Exemple 2 | Exemple 10 (20) | Exemple 3 (80) | - | 29 |
| 7 | Exemple 2 | Exemple 10 (95) | Exemple 12 (5) | - | 28 |
| 8 | Exemple 2 | Exemple 10 (80) | Exemple 3 (15) | Exemple 12 (5) | 27 |
| 9 | Exemple 2 | Exemple 10 (98) | Exemple 13 (2) | - | 28 |
| 10 | Exemple 2 | Exemple 10 (100) | - | - | 27 |
| 11 | Exemple 2 | Exemple 5 (100) | - | - | 26 |
| 12 | Exemple 2 | Exemple 5 (96) | Exemple 12 (2) | Exemple 7 (2) | 27 |
| 13 | Exemple 2 | Exemple 5 (50) | Exemple 10 (50) | - | 28 |

Les chiffres entre parenthèses indiquent le taux de couverture, par exemple pour le N°1 : la couverture est composée à 40% du composé de l'exemple 5 (ligand aminoalcool) et à 60% du composé de l'exemple 3 (composé biphosphonate non relié à un ligand).

## Revendications

1. Procédé de préparation de nanoparticules métalliques comprenant un noyau métallique N recouvert d'une couche stabilisatrice organique couplée à au moins ligand hydrophile à effet sur la stabilité/biodistribution des nanoparticules, comprenant les étapes de :
a) préparation du noyau métallique N des nanoparticules métalliques ;
b) préparation d'éléments de ciblage de formule S - C dans laquelle ;
- S est un groupe d'attache *gem*-bisphosphonate de formule X-L-CH(PO₃H₂)₂ ;
- C est un ligand de biodistribution hydrophile choisi parmi :
- les aminoalcools de formule (II) : 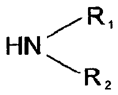 dans laquelle :
R₁ et R₂ sont identiques ou différents et représentent une chaîne hydrocarbonée aliphatique comportant de 2 à 6 atomes de carbone, substituée par 6 à 10 groupements hydroxyles, ou bien par 4 à 8 groupements hydroxyles dans le cas où R₁ et/ou R₂ est interrompu par un ou plusieurs atomes d'oxygène,
ou R₁ et R₂ représentent chacun indépendamment le groupe -(CH₂)-(CHOH)₄-CH₂OH ou - (CH₂)-CHOH-CH₂OH,
ou R₁ représente un groupe (CH₂)-(CHOH)₄-CH₂OH ou -(CH₂)-CHOH-CH₂OH tandis que R₂ représente un groupe -CH₂-(CHOH)₄-CH₂OH,
- les aminoalcools de formule (IV) : 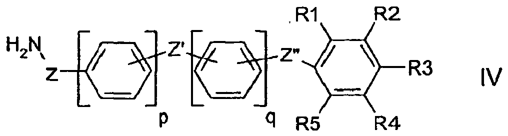 dans laquelle :
Z est une liaison, CH₂, CH₂CONH ou (CH₂)₂NHCO,
Z' est une liaison, O, S, NQ, CH₂, CO, CONQ, NQCO, NQ-CONS ou CONQCH₂CONQ,
Z" est une liaison, CONQ, NQCO ou CONQCH₂CONQ,
p et q sont des nombres entiers dont la somme vaut 0 à 3, R₁, R₂, R₃, R₄ ou R₅ représentent chacun indépendamment H, Br, CI, l, CONQ₁Q₂ ou NQ₁COQ₂,
avec Q₁ et Q₂ identiques ou différents choisis parmi H, un groupe (C₁-C₈)alkyle mono- ou polyhydroxylé et/ou éventuellement interrompu par un ou des atomes d'oxygène, de telle sorte que Q₁ et Q₂ comportent à eux deux de 4 à 10 groupes OH ;
étant entendu qu'au moins l'un et au plus deux des groupes R₁ à R₅ représente CONQ₁Q₂ ou NQ₁COQ₂;
ou bien R₁, R₃, R₅ représentent chacun indépendamment H, Br, Cl, ou I est R₂ et R₄ représentent 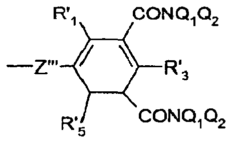
dans laquelle
R'₁, R'₃ et R'₅, identiques ou différents, représentent H, Br, Cl ou l;
Q₁ et Q₂ ont la même signification que précédemment ;
Z"' est un groupe choisi parmi CONQ, CONQCH₂CONQ, CONQCH₂, NQCONQ, CONQ(CH₂)₂NQCO; et
Q est H ou (C₁-C₄) alkyle, lesdits groupes alkyles pouvant être linéaires ou ramifiés et étant éventuellement hydroxylés,
- les polyéthylène glycols comprenant une chaîne -CH₂-(CH₂-O-CH₂)ₖ-CH₂OR₃ dans laquelle k varie de 2 à 100, et R₃ est choisi parmi H, alkyle ou -(CO)Alk, où alkyle et alk désignent un groupe aliphatique hydrocarboné, linéaire ou ramifié, ayant environ de 1 à 6 atomes de carbone dans la chaîne, ou
- les polyéthylène glycols de formule (III) : 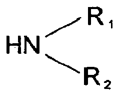 dans laquelle :
R₁ et R₂, identiques ou différents, représentent H, un groupe alkyle ou une chaîne polyéthylèneglycol de formule -CH₂-(CH₂-O-CH₂) ₖ-CH₂OR₃, étant entendu qu'au moins un des groupes R₁, R₂ représente une chaîne polyéthylèneglycol,
dans laquelle k varie de 2 à 100, et
R₃ est choisi parmi H, alkyle en C₁-C₆ ou -(CO)Alk, avec "alk" désignant un groupe alkyle en C₁-C₆;
c) greffage sur le noyau N des éléments de ciblage S-C;
avec :
● L représente un groupement organique reliant la fonction X à la fonction gem-bisphosphonate -CH(PO₃H₂)₂, L étant un groupement divalent choisi parmi :
- un groupe aliphatique éventuellement substitué par un groupe méthyle, hydroxy, méthoxy, acétoxy, amido, ou un atome de chlore, d'iode ou de brome ;
- un groupement -L₁-NHCO-L₂ où L₁ et L₂ sont soit identiques, soit différents et représentent un groupe aliphatique ; alicyclique ; aromatique ; alicyclique-aliphatique ou aromatique-aliphatique, lesdits groupes pouvant être éventuellement substitués par un groupe méthyle, hydroxy, méthoxy, acétoxy, amido ou un atome de chlore, d'iode ou de brome;
● X représente une fonction chimique capable d'être couplée au ligand hydrophile C et est choisie parmi :
- -COOH,
- -NH₂, -NCS, -NH-NH₂, -CHO, alkylpyrocarbonyle (-CO-O-CO-alk), acylazidyle (-CO-N₃), iminocarbonate (-O-C(NH)-NH₂), vinylsulfuryle (-S-CH=CH₂), pyridyldisulfuryle (-S-S-Py), haloacétyle, maléimidyle, dichlorotriazinyle, halogène,
- le groupe de formule:

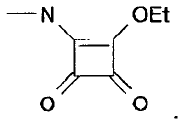

2. Procédé selon la revendication 1, dans lequel le noyau métallique est choisi parmi les suivants : hydroxyde de fer, oxyde de fer hydraté, ferrite, oxyde de fer mixte.

3. Procédé selon la revendication 1 ou 2, dans lequel le ligand hydrophile de biodistribution est un ligand aminoalcool.

4. Procédé selon la revendication 1 ou 2, dans lequel le ligand hydrophile de biodistribution est un ligand polyéthylène glycol.

5. Procédé selon la revendication 1 à 4, dans lequel une partie des ligands de biodistribution hydrophile C sont des ligands aminoalcool, et une autre partie sont des ligands polyéthylène glycol.

6. Procédé selon la revendication 1 à 5, dans lequel l'on greffe sur le noyau d'une part des éléments de ciblage S-C, et d'autre part des groupes de stabilisation S non porteurs de ligands de biodistribution.

7. Procédé selon la revendication 1 à 6, dans lequel le taux de greffage des éléments de ciblage sur le noyau est de 1 à 10%, avantageusement 1, 2, 3, 5, 10 %.

8. Procédé selon l'une quelconque des revendications 1 à 7, comprenant en outre le greffage d'éléments S-C-T, où C est un ligand polyéthylèneglycol et T représente un groupe chromophore.

## Claims

1. Process for preparing metallic nanoparticles comprising a metallic core N covered with an organic stabilizing layer coupled to at least one hydrophilic ligand having an effect on the stability/biodistribution of the nanoparticles, comprising the steps of:
a) preparing the metallic core N of the metallic nanoparticles;
b) preparing targeting elements of formula S-C in which:
- S is a gem-bisphosphonate attachment group of formula X-L-CH(PO₃H₂)₂;
- C is a hydrophilic biodistribution ligand chosen from:
- amino alcohols of formula (II): 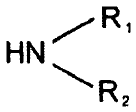 in which:
R₁ and R₂ are identical or different and represent an aliphatic hydrocarbon-based chain comprising from 2 to 6 carbon atoms, substituted with 6 to 10 hydroxyl groups, or else with 4 to 8 hydroxyl groups in the case where R₁ and/or R₂ is interrupted by one or more oxygen atoms,
or R₁ and R₂ each independently represent the group -(CH₂)-(CHOH)₄-CH₂OH or -(CH₂)-CHOH-CH₂OH,
or R₁ represents a group (CH₂)-(CHOH)₄-CH₂OH or -(CH₂)-CHOH-CH₂OH while R₂ represents a group -CH₂-(CHOH)₄-CH₂OH,
- amino alcohols of formula (IV): 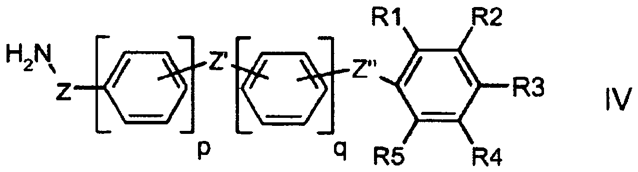 in which:
Z is a bond, CH₂, CH₂CONH or (CH₂)₂NHCO;
Z' is a bond, O, S, NQ, CH₂, CO, CONQ, NQCO, NQ-CONQ or CONQCH₂CONQ,
Z" is a bond, CONQ, NQCO or CONQCH₂CONQ;
p and q are integers, the sum of which is equal to 0 to 3;
R₁, R₂, R₃, R₄ or R₅ each independently represent H, Br, Cl, I, CONQ₁Q₂ or NQ₁COQ₂;
with Q₁ and Q₂, which are identical or different, chosen from H, a (C₁-C₈)alkyl group that is monohydroxylated or polyhydroxylated and/or optionally interrupted by one or more oxygen atoms, so that Q₁ and Q₂ comprise, between them, from 4 to 10 OH groups;
it being understood that at least one and at most two of the groups R₁ to R₅ represents CONQ₁Q₂ or NQ₁COQ₂;
or else R₁, R₃, R₅ each independently represent H, Br, Cl or I and R₂ and R₄ represent: 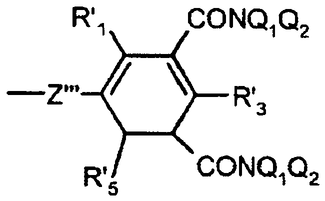 in which:
R'₁, R'₃ and R'₅, which are identical or different, represent H, Br, Cl or I;
Q₁ and Q₂ have the same meaning as above; Z"' is a group chosen from CONQ, CONQCH₂CONQ, CONQCH₂, NQCONQ, CONQ(CH₂)₂NQCO; and
Q is H or (C₁-C₄)alkyl, said alkyl groups possibly being linear or branched and optionally being hydroxylated,
- polyethylene glycols comprising a -CH₂-(CH₂-O-CH₂)ₖ-CH₂OR₃ chain in which k varies from 2 to 100, and R₃ is chosen from H, alkyl or -(CO)Alk, where alkyl and alk denote an aliphatic, linear or branched, hydrocarbon-based group having around 1 to 6 carbon atoms in the chain, or
- polyethylene glycols of formula (III): 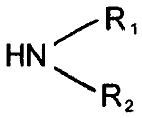 in which:
R₁ and R₂, which are identical or different, represent H, an alkyl group or a polyethylene glycol chain of formula -CH₂-(CH₂-O-CH₂)ₖ-CH₂OR₃, it being understood that at least one of the groups R₁, R₂ represents a polyethylene glycol chain;
in which k varies from 2 to 100; and
R₃ is chosen from H, C₁-C₆ alkyl or -(CO)Alk, with "alk" denoting a C₁-C₆ alkyl group;
c) grafting of the targeting elements S-C to the core N;
where:
● L represents an organic group that connects the X functional group to the gem-bisphosphonate - CH(PO₃H₂)₂ functional group, L being a divalent group chosen from:
- an aliphatic group optionally substituted with a methyl, hydroxy, methoxy, acetoxy or amido group or a chlorine, iodine or bromine atom;
- an -L₁-NHCO-L₂ group where L₁ and L₂ are either identical or different and represent an aliphatic; alicyclic; aromatic; alicyclic-aliphatic or aromatic-aliphatic group, said groups possibly being optionally substituted with a methyl, hydroxy, methoxy, acetoxy or amido group or a chlorine, iodine or bromine atom;
● X represents a chemical functional group capable of being coupled to the hydrophilic ligand C and is chosen from:
- -COOH,
- -NH₂, -NCS, -NH-NH₂, -CHO, alkylpyrocarbonyl (-CO-O-CO-alk), acylazidyl (-CO-N₃), iminocarbonate (-O-C(NH)-NH₂), vinylsulfuryl (-S-CH=CH₂), pyridyldisulfuryl (-S-S-Py), haloacetyl, maleimidyl, dichlorotriazinyl, halogen,
- the group of formula:

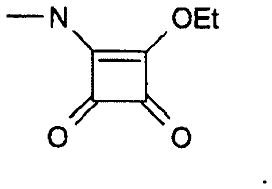

2. Process according to Claim 1, in which the metallic core is chosen from the following: iron hydroxide, hydrated iron oxide, ferrite, or mixed iron oxide.

3. Process according to Claim 1 or 2, in which the hydrophilic biodistribution ligand is an amino alcohol ligand.

4. Process according to Claim 1 or 2, in which the hydrophilic biodistribution ligand is a polyethylene glycol ligand.

5. Process according to Claims 1 to 4, in which one portion of the hydrophilic biodistribution ligands C are amino alcohol ligands and another portion are polyethylene glycol ligands.

6. Process according to Claims 1 to 5, in which grafted to the core are, on the one hand, targeting elements S-C and, on the other hand, stabilizing groups S that do not bear biodistribution ligands.

7. Process according to Claims 1 to 6, in which the degree of grafting of the targeting elements to the core is from 1 to 10%, advantageously 1, 2, 3, 5 or 10%.

8. Process according to any one of Claims 1 to 7, comprising, in addition, the grafting of elements S-C-T, where C is a polyethylene glycol ligand and T represents a chromophore group.

## Patentansprüche

1. Verfahren zur Herstellung von metallischen Nanopartikeln, umfassend einen metallischen Kern N, der mit einer organischen stabilisierenden Schicht bedeckt ist, die mit mindestens einem hydrophilen Liganden mit Wirkung auf die Stabilität/Bioverteilung der Nanopartikel gekoppelt ist, umfassend die folgenden Schritte:
a) Herstellung des metallischen Kerns N der metallischen Nanopartikel;
b) Herstellung von Ansteuerungselementen der Formel S-C, worin:
- S für eine gem-Bisphosphonat-Anbindungsgruppe der Formel X-L-CH(PO₃H₂)₂ steht;
- C für einen hydrophilen Bioverteilungsliganden steht, der aus
- Aminoalkoholen der Formel (II): 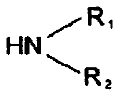 worin R₁ und R₂ gleich oder verschieden sind und für eine aliphatische Kohlenwasserstoffkette mit 2 bis 6 Kohlenstoffatomen, die vorzugsweise durch 6 bis 10 Hydroxylgruppen oder auch durch 4 bis 8 Hydroxylgruppen substituiert ist, wenn R₁ und/oder R₂ durch ein oder mehrere Sauerstoffatome unterbrochen ist, stehen oder R₁ und R₂ jeweils unabhängig für die Gruppe -(CH₂) -(CHOH)₄ -CH₂OH oder -(CH₂)-CHOH-CH₂OH stehen
oder R₁ für eine Gruppe (CH₂)-(CHOH)₄-CH₂OH oder -(CH₂)-CHOH-CH₂OH steht, während R₂ für eine Gruppe -(CH₂)-(CHOH)₄-CH₂OH steht,
- Aminoalkoholen der Formel (IV): 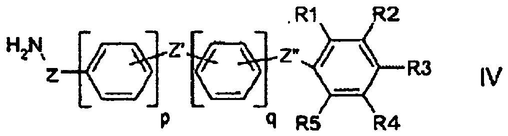 worin:
Z für eine Bindung, CH₂, CH₂CONH oder (CH₂)₂NHCO steht,
Z' für eine Bindung, O, S, NQ, CH₂, CO, CONQ, NQCO, NQ-CONQ oder CONQCH₂CONQ steht, Z" für eine Bindung, CONQ, NQCO oder CONQCH₂CONQ steht,
p und q für ganze Zahlen stehen, deren Summe 0 bis 3 beträgt,
R₁, R₂, R₃, R₄ oder R₅ jeweils unabhängig für H, Br, Cl, I, CONQ₁Q₂ oder NQ₁COQ₂ stehen,
wobei Q₁ und Q₂ gleich oder verschieden sind und aus H und einer (C₁-C₈) -Alkylgruppe, die mono- oder polyhydroxyliert und/oder gegebenenfalls durch ein oder mehrere Sauerstoffatome unterbrochen ist, so dass Q₁ und Q₂ zusammen 4 bis 10 OH-Gruppen umfassen, ausgewählt sind;
mit der Maßgabe, dass mindestens eine und höchstens zwei der Gruppen R₁ bis R₅ für CONQ₁Q₂ oder NQ₁COQ₂ stehen;
oder R₁, R₃ und R₅ jeweils unabhängig für H, Br, Cl oder I stehen und R₂ und R₄ für 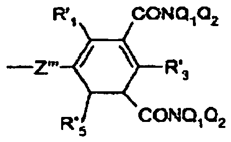 stehen, worin
R'₁, R'₃ und R'₅ jeweils unabhängig für H, Br, Cl oder I stehen;
Q₁ und Q₂ die gleiche Bedeutung wie oben besitzen;
Z"' für eine aus CONQ, CONQCH₂CONQ, CONQCH₂, NQCONQ und CONQ(CH₂)₂NQCO ausgewählte Gruppe steht und
Q für H oder (C₁-C₄)-Alkyl steht, wobei die Alkylgruppen linear oder verzweigt und gegebenenfalls hydroxyliert sind,
- Polyethylenglykolen mit einer -CH₂-(CH₂-O-CH₂)ₖ-CH₂OR₃-Kette, worin k im Bereich von 2 bis 100 liegt und R₃ aus H, Alkyl oder -(CO)Alk ausgewählt ist, wobei Alkyl und Alk für eine lineare oder verzweigte aliphatische Kohlenwasserstoffgruppe mit ungefähr 1 bis 6 Kohlenstoffatomen in der Kette stehen, oder
- Polyethylenglykolen der Formel (III): 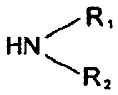 worin:
R₁ und R₂ gleich oder verschieden sind und für H, eine Alkylgruppe oder eine Polyethylenglykolkette der Formel -CH₂-(CH₂-O-CH₂)ₖ-CH₂OR₃ stehen, mit der Maßgabe, dass mindestens eine der Gruppen R₁ und R₂ für eine Polyethylenglykolkette steht,
worin k im Bereich von 2 bis 100 liegt und R₃ aus H, C₁-C₆-Alkyl oder - (CO) Alk ausgewählt ist, wobei "Alk" für eine C₁-C₆-Alkylgruppe steht,
ausgewählt ist;
c) Aufpfropfen der Ansteuerungselemente S-C auf den Kern N;
wobei:
● L für eine organische Gruppierung steht, die die Funktion X mit der *gem*-Bisphosphonat-Funktion -CH(PO₃H₂)₂ verbindet, wobei L für eine zweiwertige Gruppierung steht, die aus:
- einer aliphatischen Gruppe, die gegebenenfalls durch eine Methyl-, Hydroxy-, Methoxy-, Acetoxy- oder Amidogruppe oder ein Chlor-, Iod- oder Bromatom substituiert ist;
- einer Gruppierung -L₁-NHCO-L₂, wobei L₁ und L₂ gleich oder verschieden sind und für eine aliphatische, alicyclische, aromatische, alicyclisch-aliphatische oder aromatischaliphatische Gruppe stehen, wobei diese Gruppen gegebenenfalls durch eine Methyl-, Hydroxy-, Methoxy-, Acetoxy- oder Amidogruppe oder ein Chlor-, Iod- oder Bromatom substituiert sein können;
ausgewählt ist;
● X für eine chemische Funktion steht, die an den hydrophilen Liganden C gekoppelt werden kann und aus:
- -COOH,
- -NH₂, -NCS, -NH-NH₂, -CHO, Alkylpyrocarbonyl (-CO-O-CO-Alk), Acylazidyl (-CO-N₃), Iminocarbonat (-O-C(NH)-NH₂), Vinylsulfuryl (-S-CH=CH₂), Pyridyldisulfuryl (-S-S-Py), Halogenacetyl, Maleinimidyl, Dichlortriazinyl, Halogen,
- der Gruppe der Formel: 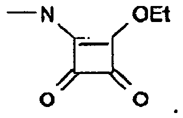
ausgewählt ist.

2. Verfahren nach Anspruch 1, bei dem man den metallischen Kern unter den Folgenden auswählt: Eisenhydroxid, Eisenoxidhydrat, Ferrit, Eisenmischoxid.

3. Verfahren nach Anspruch 1 oder 2, bei dem es sich bei dem hydrophilen Bioverteilungsliganden um einen Aminoalkohol-Liganden handelt.

4. Verfahren nach Anspruch 1 oder 2, bei dem es sich bei dem hydrophilen Bioverteilungsliganden um einen Polyethylenglykol-Liganden handelt.

5. Verfahren nach einem der Ansprüche 1 bis 4, bei dem es sich bei einem Teil der hydrophilen Bioverteilungsliganden C um Aminoalkohol-Liganden und bei einem anderen Teil um Polyethylenglykol-Liganden handelt.

6. Verfahren nach einem der Ansprüche 1 bis 5, bei dem man auf den Kern zum einen Ansteuerungselemente S-C und zum anderen Stabilisierungsgruppen S, die keine Bioverteilungsgruppen tragen, aufpfropft.

7. Verfahren nach einem der Ansprüche 1 bis 6, bei dem der Pfropfgrad der Ansteuerungselemente auf den Kern 1 bis 10% und vorteilhafterweise 1, 2, 3, 5, 10% beträgt.

8. Verfahren nach einem der Ansprüche 1 bis 7, das außerdem das Aufpfropfen von Elementen S-C-T umfasst, wobei C für einen Polyethylenglykol-Liganden steht und T für eine Chromophorgruppe steht.
